# EUROPÄISCHE PATENTSCHRIFT

(11) **EP 1 911 485 B1**
(45) Veröffentlichungstag und Bekanntmachung des Hinweises auf die Patenterteilung: **09.09.2009**
(21) Anmeldenummer: 08000580.4
(22) Anmeldetag: 02.07.2003
(51) Int. Cl.: A61M 25/06

(54) **Kathetereinführvorrichtung**
Catheter insertion device
Dispositif d'introduction de cathéter

(30) Priorität: 04.07.2002 DE 20210394 U
(43) Veröffentlichungstag der Anmeldung: 16.04.2008
(62) Teilanmeldung aus: 03762595.1
(73) Patentinhaber: B. BRAUN MELSUNGEN AG, 34212 Melsungen (DE)
(72) Erfinder: Woehr, Kevin, 34587 Felsburg (DE)
(74) Vertreter: Klingseisen, Franz

(56) Entgegenhaltungen:
- EP-A- 1 003 588
- DE-A- 3 210 148
- DE-A- 4 434 569
- DE-U- 20 104 539

## Beschreibung

Die Erfindung betrifft eine Kathetereinführvorrichtung nach dem Oberbegriff des Anspruchs 1.

Eine Vorrichtung dieser Art ist aus DE 44 34 569A bekannt, bei der eine scheibenförmige Dichtung in einem rohrförmigen Ansatz in einen hohlzylindrischen Abschnitt eines Bauteils eingreift, das in die Katheterbuchse eingesetzt ist. Ein Teil des Bauteils liegt außerhalb der Katheterbuchse und ist mit einem Nadelschutzelement versehen. Bei einer Bauform mit Ventilscheibe ist diese stirnseitig an dem Bauteil angebracht. Beim Herausziehen der Nadel aus der Katheterbuchse wird das Nadelschutzelement zusammen mit dem Bauteil von der Katheterbuchse entfernt, sodass die scheibenförmige Dichtung aus der Katheterbuchse mit herausbewegt wird. Hierdurch kann Blut aus der Katheterbuchse austreten. Dadurch, dass das Nadelschutzelement in der Bereitstellung außerhalb der Katheterbuchse angeordnet ist, ergibt sich ein in Achsrichtung sperriger Aufbau.

Weitere Vorrichtungen sind aus DE 201 04 539 U und EP 1 003 588 bekannt, wobei in einer hohlen Katheterbuchse ein Nadelschutzelement angeordnet ist, das beim Zurückziehen der Hohlnadel aus dem Katheter über eine Eingriffseinrichtung nahe der Spitze der Hohlnadel mit dieser in Eingriff tritt und die Spitze abdeckt, wenn die Hohlnadel vom Katheter getrennt wird. Bei dieser Ausgestaltung kann nach dem Herausziehen der Hohlnadel aus dem Katheter durch diesen Blut austreten, mit dem das Bedienungspersonal in Kontakt kommen kann.

Der Erfindung liegt die Aufgabe zugrunde, eine Kathetereinführvorrichtung der eingangs angegebenen Art so auszubilden, dass ein Blutaustritt aus dem Katheter nach dem Entfernen der Hohlnadel mit Nadelschutzelement verhindert wird.

Diese Aufgabe wird erfindungsgemäß durch die Merkmale im kennzeichnenden Teil des Anspruchs 1 gelöst. Dadurch, dass in der Bereitstellung zwischen Katheter und Nadelschutzelement ein Rückschlagventil in der Katheterbuchse angeordnet ist, durch das sich die Hohlnadel erstreckt, kann nach dem Herausziehen der Hohlnadel aus dem Katheter dieser zuverlässig abgeschlossen werden, so dass ein Blutaustritt verhindert wird, während gleichzeitig die Spitze der Hohlnadel durch das Nadelschutzelement sicher abgedeckt wird, so dass sich die Bedienungsperson an der Nadelspitze nicht verletzen kann.

Eine beispielsweise Ausführungsform der Erfindung wird nachfolgend anhand der Zeichnung näher erläutert. Es zeigen
- Fig. 1: einen Längsschnitt durch eine Kathetereinführvorrichtung einer anderen Bauform,
- Fig. 2: die Kathetereinführvorrichtung von Fig. 1 mit herausgezogener Hohlnadel,
- Fig. 3: eine erfindungsgemäße Ausführungsform im Längsschnitt, und
- Fig. 4: Stirnansichten des Ventilbetätigungselementes von Fig. 3,

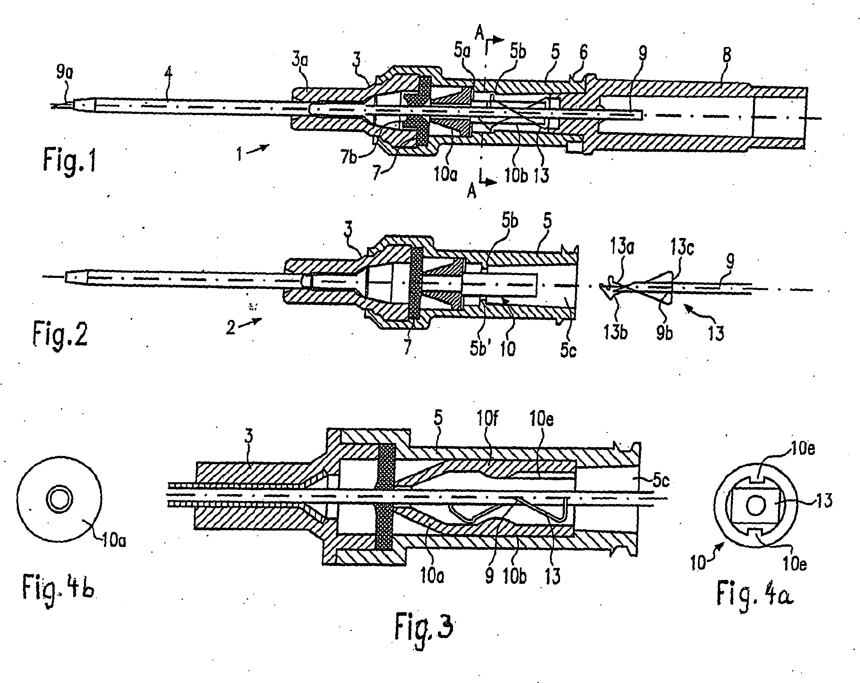

Fig. 1 zeigt eine Kathetereinführvorrichtung 1 mit einer Katheterbuchse 2, die bei dem dargestellten Ausführungsbeispiel zweiteilig ausgebildet ist. Ein distales Buchsenelement 3 der Katheterbuchse weist einen Halteabschnitt 3a auf, in dem ein Katheter 4 eingepresst ist. Das proximale Ende des Buchsenelementes 3 ist gegenüber dem distalen Ende im Durchmesser vergrößert und bildet einen Verbindungsabschnitt mit einem Buchsenelement 5, das mit dem distalen Ende das proximale Ende des Buchsenelements 3 übergreift und am proximalen Ende mit einem Luer-Gewinde 6 versehen ist. Zwischen den beiden Buchsenelementen 3 und 5 ist ein Rückschlagventil in Form einer Ventilscheibe 7 eingesetzt, die durch die beiden Buchsenelemente 3 und 5 in ihrer Lage fixiert ist.

In der Bereitstellung nach Fig. 1 ist in die Katheterbuchse 2 eine Nadelbuchse 8 eingesetzt, an der eine Hohlnadel 9 befestigt ist, die sich durch die Ventilscheibe 7 und den Katheter 4 erstreckt, so dass die Nadelspitze 9a frei liegt. Zwischen Nadelbuchse 8 und Ventilscheibe 7 ist im proximalen Buchsenelement 5 ein Ventilbetätigungselement 10 verschiebbar angeordnet, das einen kegelstumpfförmigen Anlageabschnitt 10a aufweist, der zum Öffnen der Ventilscheibe 7 dient. Auf der proximalen Seite schließt sich an den Anlageabschnitt 10a ein Stößelabschnitt 10b an, der einen Hohlraum zur Aufnahme eines Nadelschutzelementes 13 aufweist. Bei dem dargestellten Ausführungsbeispiel ist der Stößelabschnitt 10b durch zwei beabstandete Stößel ausgebildet, zwischen denen das Nadelschutzelement in Form eines Federclips 13 eingesetzt ist.

Wenn die Hohlnadel 9 aus der Katheterbuchse 2 zurückgezogen wird, kommt eine nahe der Nadelspitze 9a vorgesehene Eingriffseinrichtung 9b (Fig. 2) in Form eines radialen Vorsprungs an der Hohlnadel, der durch eine leichte Quetschung ausgebildet sein kann, mit dem Außenumfang einer Bohrung in der Rückwand 13c des Federclips 13 in Eingriff, so dass der Federclip 13 mit der Nadel 9 aus der Katheterbuchse gezogen wird, während sich gleichzeitig die Federarme 13a und 13b des Federclips um die Nadelspitze legen und diese vollständig abdecken und blockieren. In dieser in Fig. 2 wiedergegebenen Trennstellung schließt die Ventilscheibe 7 aufgrund ihrer Elastizität die Durchtrittsöffnung für die Hohlnadel 9, so dass durch den Katheter 4 kein Blut austreten kann. Die Ventilscheibe ist beispielsweise mit drei von der Mitte ausgehenden und sich radial über einen kurzen Abschnitt erstreckenden Einschnitten versehen, die elastische Laschen dazwischen bilden, welche durch die Hohlnadel aufgeweitet werden können.

Fig. 3 zeigt eine erfindungsgemäße Ausführungsform mit einem hohlzylindrischen Ventilbetätigungselement 10, auf dessen Innenumfang ein Wulst 10f zur Positionierung des Federclips 13 innerhalb des Ventilbetätigungselementes 10 ausgebildet ist. Fig. 4a zeigt eine Stirnansicht des Ventilbetätigungselementes 10 von rechts und Fig. 4b eine Stirnansicht von links in Fig. 3, wobei für die Anlage des Halsabschnittes 14a einer Spritze 14 bei dieser Ausführungsform radial nach innen vorstehende Rippen 10e ausgebildet sind, die radial in die Bohrung 5c des Buchsenelementes 5 hinein ragen, wie dies die obere Hälfte des Ventilbetätigungselementes in Fig. 3 zeigt, in der die Schnittansicht der unteren Hälfte des Ventilbetätigungselementes 10 um 90° verdreht zur oberen Hälfte wiedergegeben ist.

Bei der Ausführungsform einer Kathetereinführvorrichtung nach Fig. 3 kann die Ventilscheibe 7 in der Stellung des Ventilbetätigungselementes 10 entsprechend Fig. 2 durch einen mittels der Spritze erzeugten Unterdruck zum Absaugen von Flüssigkeit aus dem Katheter geöffnet werden, wobei die elastischen Laschen durch den Unterdruck aufgebogen werden.

Das Rückschlagventil in Form einer Ventilscheibe 7 wird zweckmäßigerweise aus elastischem Silikon gefertigt, während für die Buchsenelemente 3 und 5 sowie für das Ventilbetätigungselement 10 ein entsprechend steifer Kunststoff verwendet wird.

## Patentansprüche

1. Kathetereinfühüvorrichtung, umfassend
eine etwa hohlzylindrische Katheterbuchse (2), an deren distalem Ende ein Katheter (4) angebracht ist,
eine Nadelbuchse (8) mit einer daran angebrachten Hohlnadel (9), die sich in der Bereitstellung durch die Katheterbuchse (2) und den Katheter (4) erstreckt,
ein Rückschlagventil (7), durch das sich die Hohlnadel (9) in der Bereitstellung erstreckt und das sich nach Herausziehen der Nadel selbsttätig schließt, und
ein Nadelschutzelement (13), das auf der Nadel (9) verschiebbar ist und mit der Nadel nahe der Nadelspitze in Eingriff tritt, wenn die Hohlnadel aus der Katheterbuchse (2) herausgezogen wird,
**gekennzeichnet durch**
ein Ventilbetätigungselement (10), das in der Katheterbuchse (2) verschiebbar geführt und proximal von dem in der Katheterbuchse (2) angeordneten Rückschlagventil (7) angeordnet ist sowie einen hohlzylindrischen Raum zur Aufnahme der Nadel (9) aufweist,
wobei das Nadelschutzelement (13) in dem hohlzylindrischen Raum des Ventilbetätigungselements (10) angeordnet ist und einen Eingriffsabschnitt aufweist, der mit einer auf dem Innenumfang des Ventilbetätigungselements (10) vorgesehenen Eingriffseinrichtung (10f) in Eingriff tritt, wenn die Hohlnadel (9) aus der Katheterbuchse (2) herausgezogen wird.

2. Vorrichtung nach Anspruch 1, wobei die Katheterbuchse (2) zweiteilig ausgebildet ist und das Rückschlagventil (7) zwischen einem distalen Buchsenelelement (3) und einem proximalen Buchsenelement (5) gehalten ist, die miteinander verbunden sind.

3. Vorrichtung nach Anspruch 1, wobei das Ventilbetätigungselement (10) als Hohlzylinder mit einem kegelstumpffönnigen distalen Endabschnitt (10a) ausgebildet ist.

4. Vorrichtung nach Anspruch 1, wobei die Eingriffseinrichtung auf dem Innenumfang des Ventilbetätigungselements (10) in der Form eines radialen Vorsprungs (10f) ausgebildet ist.

5. Vorrichtung nach Anspruch 1, wobei das Rückschlagventil (7) in der Form einer Ventilscheibe mit wenigstens einem radialen Schlitz (7a) ausgebildet ist, der sich von der Mitte der Ventilscheibe aus radial wach außen erstreckt.

6. Vorrichtung nach einem der vorhergehenden Ansprüche, wobei die Nadel (9) eine Eingriffseinrichtung für das Nadelschutzelement (13) in Form eines radialen Vorsprungs (9b) aufweist.

7. Vorrichtung nach Anspruch, 6, wobei das Nadelschutzelement als Federclip (13) ausgebildet ist, der ausgehend von einer mit einer Bohrung versehenen Rückwand (13c) diametral gegenüberliegende Federarme (13a, 13b) aufweist, wobei die Federarme einander kreuzen und in der Bereitstellung durch die Nadel auseinandergedrückt werden, und wobei distale Endabschnitte die Nadelspitze übergreifen und blockieren, wenn der radiale Vorsprung (9b) der Nadel an der Rückwand (13c) des Nadelschutzelementes (13) zum Anliegen kommt, wenn die Nadel aus dem Katheter zurückgezogen wird.

## Claims

1. Catheter insertion device comprising
an approximately hollow-cylindrical catheter hub (2) to whose distal end a catheter (4) is attached,
a needle hub (8) having a hollow needle (9) attached thereon and extending in the ready position through the catheter hub (2) and the catheter (4),
a check valve (7) through which the hollow needle (9) extends in the ready position and which automatically closes after the needle is removed, and
a needle guard element (13) arranged displaceably on the needle (9) and engaging with the needle near the needle tip when the hollow needle is removed from the catheter hub (2),
**characterized by**
a valve actuating element (10) displaceably guided in the catheter hub (2) and arranged proximally of the check valve (7) arranged in the catheter hub (2) and having a hollow cylindrical space for receiving the needle (9),
wherein the needle guard element (13) is arranged in the hollow cylindrical space of the valve actuating element (10) and has an engaging section which engages with an engaging means (10f) provided on the inner circumference of the valve actuating element (10) when the hollow needle (9) is removed from the catheter hub (2).

2. Device according to claim 1, wherein the catheter hub (2) has a two-part form and the check valve (7) is held between a distal hub element (3) and a proximal hub element (5) which are joined to one another.

3. Device according to claim 1, wherein the valve actuating element (10) is formed as a hollow cylinder with a truncated cone-shaped distal end section (10a).

4. Device according to claim 1, wherein the engaging means is formed on the inner circumference of the valve actuating element (10) in the shape of a radial projection (10f).

5. Device according to claim 1, wherein the check valve (7) is formed in the shape of a valve disc with at least one radial slit (7a) extending from the centre of the valve disc radially outward.

6. Device according to one of the preceding claims, wherein the needle (9) has an engaging means for the needle guard element (13) in the shape of a radial projection (9b).

7. Device according to claim 6, wherein the needle guard element is formed as a spring clip (13) which has diametrically opposite spring arms (13a, 13b) starting from a rear wall (13c) provided with a bore, wherein the spring arms cross each other and, in the ready position, are displaced apart by the needle, and wherein distal end sections overlap and block the needle tip when the radial projection (9b) of the needle comes to abut on the rear wall (13c) of the needle guard element (13) when the needle is removed from the catheter.

## Revendications

1. Dispositif d'introduction de cathéter, comprenant
un connecteur de cathéter à peu près cylindrique creux (2) à l'extrémité distale duquel un cathéter (4) est fourni,
un connecteur d'aiguille (8), doté d'une aiguille creuse (9) fournie à celui-ci, qui s'étend dans une mise en service, en passant par le connecteur de cathéter (2) et le cathéter (4),
un clapet anti-retour (7) dans lequel s'étend l'aiguille creuse (9) dans la mise en service et qui se ferme automatiquement après retrait de l'aiguille, et
un élément protecteur de l'aiguille (13) qui est déplaçable sur l'aiguille (9) et est en prise avec l'aiguille à proximité de la pointe d'aiguille, lorsque l'aiguille creuse est retirée du connecteur de cathéter (2),
**caractérisé par**
un élément d'actionnement de soupape (10) qui est conduit de façon à pouvoir être déplacé dans le connecteur du cathéter (2) et est disposé au niveau proximal du clapet anti-retour (7) disposé dans le connecteur du cathéter et présente un espace cylindrique creux pour recevoir l'aiguille (9),
l'élément protecteur de l'aiguille (13) étant disposé dans l'espace cylindrique creux de l'élément d'actionnement de soupape (10) et présente un segment de mise en prise qui est en prise avec un dispositif de mise en prise (10f) prévu sur le pourtour intérieur de l'élément d'actionnement de soupape (10) lorsque l'aiguille creuse (9) est retirée du connecteur de cathéter (2).

2. Dispositif selon la revendication 1, dans lequel le connecteur du cathéter (2) est constitué de deux parties et le clapet anti-retour (7) est maintenu entre un élément de connecteur distal (3) et un élément de connecteur proximal (5), qui sont reliés l'un à l'autre.

3. Dispositif selon la revendication 1, dans lequel l'élément d'actionnement de soupape (10) est constitué sous la forme d'un cylindre creux présentant un segment terminal distal en forme de cône tronqué.

4. Dispositif selon la revendication 1, dans lequel le dispositif de mise en prise est constitué sur le pourtour intérieur de l'élément d'actionnement de soupape (10) sous la forme d'une proéminence radiale (10f).

5. Dispositif selon la revendication 1, dans lequel le clapet anti-retour (7) est constitué sous la forme d'un disque de soupape présentant au moins une fente radiale (7a) qui s'étend du milieu du disque de soupape au niveau radial vers l'extérieur.

6. Dispositif selon l'une des revendications précédentes, dans lequel l'aiguille (9) présente un dispositif de mise en prise pour l'élément protecteur de l'aiguille (13) sous la forme d'une proéminence radiale (9b).

7. Dispositif selon la revendication 6, dans lequel l'élément protecteur de l'aiguille est constitué comme un clip à ressort (13) qui présente à partir d'une paroi arrière (13c) dotée d'un alésage, des bras de ressort diamétralement opposés (13a, 13b), les bras de ressort se croisant et étant comprimés dans le sens d'une séparation par l'aiguille dans la mise en service, et des segments terminaux distaux recouvrant la pointe de l'aiguille et la bloquant, lorsque la proéminence radiale (9b) de l'aiguille s'appuie sur la paroi arrière (13c) de l'élément protecteur de l'aiguille (13), lorsque l'aiguille est retirée du cathéter.
